# EUROPÄISCHE PATENTSCHRIFT

(11) **EP 2 027 829 B1**
(45) Veröffentlichungstag und Bekanntmachung des Hinweises auf die Patenterteilung: **02.09.2015**
(21) Anmeldenummer: 08011996.9
(22) Anmeldetag: 03.07.2008
(51) Int. Cl.: A61C 1/08, A61C 8/00

(54) **Bohrerführung mit einem Anschlag**
Drill guide with a stop
Glissière de foret pourvue d'une butée

(30) Priorität: 22.08.2007 EP 07016398
(43) Veröffentlichungstag der Anmeldung: 25.02.2009
(73) Patentinhaber: Straumann Holding AG, 4002 Basel (CH)
(72) Erfinder: Suter, Edmund, 4435 Niederdorf (CH); Streff, Patrick, 79576 Weil am Rhein (DE); Kühne, Steffen, 4313 Möhlin (CH)
(74) Vertreter: Schaad, Balass, Menzl & Partner AG

(56) Entgegenhaltungen:
- WO-A-94/00073
- US-A- 5 888 034
- US-A1- 2006 240 378

## Beschreibung

Die vorliegende Erfindung betrifft eine Bohrerführung mit einem Anschlag zur Verwendung im Dentalbereich.

Zum Erzielen einer prothetisch optimierten Implantatachse wird häufig eine Bohrschablone, z.B. eine OP-Schablone/CT-Schiene eingesetzt. Bekannt sind Bohrschablonen zum Führen von Dentalimplantatbohrern zur Aufbereitung der Kieferknochen, welche zur Aufnahme eines oder mehreren Dentalimplantaten dienen sollen. Derartige Bohrschablonen werden manuell modelliert oder computergesteuert hergestellt (CAM). Sie weisen Bohrlöcher auf, die zur Führung eines Dentalimplantatbohrers oder des Implantates während eines operativen Eingriffs dienen. Oft werden in die Bohrschablonen standardisierte Metallbohrhülsen (nachfolgend Standardbohrhülsen) eingearbeitet, z.B. einpolymerisiert oder eingepresst, um die Präzision der Bohrungen zu erhöhen. Während eines operativen Eingriffs, wird der Dentalimplantatbohrer durch eine derartige Hülse geführt. Die Bohrschablone dient somit dazu, die Bohrung präzise gemäss den aus der Planung ermittelten optimalen Implantatachsen umzusetzen.

Bei den meisten Implantationsverfahren werden die Bohrungen in mehreren Schritten erarbeitet. Beispielsweise erfolgt eine erste Bohrung mit einem Pilotbohrer geringen Durchmessers, gefolgt von einer Bohrung mit einem Spiralbohrer mit dem definitiven Durchmessers des zu setzenden Implantats. Es können je nach Verfahren auch weitere Bohrer mit Zwischendurchmessern oder anderen Schneidgeometrien erforderlich sein.

Um dieses Verfahren anzuwenden, werden entsprechende Reduktionshülsen, die den Durchmesser der Bohrung definieren, in die Löcher oder Standardbohrhülsen der Bohrschablonen eingelegt.

Da die Reduktionshülsen auch während der Behandlung ausgetauscht werden und diese typischerweise Durchmesser von weniger als 6mm haben, ergeben sich Schwierigkeiten bei der Handhabung oder gar die Gefahr der Aspiration durch den Patienten.

Ein weiteres Problem ist, dass der Bohrer nicht zu tief in den Knochen eindringen darf, da ansonsten die Nerven geschädigt werden können.

Aus WO 06/130067 sind Reduktionshülsen mit griffartigen Verlängerungen bekannt. Die schrittweise Durchmesserreduktion wird durch Ineinanderstapeln mehrerer Reduktionshülsen erreicht.

In WO 06/014130 wird eine Reduktionshülse beschrieben, die über ein Kugelgelenk mit einem Griffteil verbunden ist, um verschiedenen Platzsituationen im Patientenmund gerecht werden zu können.

WO 97/49351 offenbart eine implantatgestützte Vorrichtung zur Bohrerführung mit einem Hilfseinsatz, aus dem zwei Reduktionshülsen gleichen Durchmessers ragen, die über eine flache Verbindung in paralleler Position gehalten werden.

US 5,888,034 beschreibt eine Bohrerführung mit einer an einem Griffteil angebrachten Führungshülse, deren Länge teleskopartig eingestellt werden kann. In dem Griff befinden sich weitere Reduktionshülsen. Die manuelle Einstellung der gewünschten Bohrtiefe ist zeitintensiv und verlängert die Behandlungszeit. Zudem ist die beschriebene Bohrerführung schwer sterilisierbar.

WO 94/00073 offenbart eine Vorrichtung zum Führen von Implantaten mit einem Befestigungsmittel und einer Bohrerführung, die relativ zum Befestigungsmittel bewegbar ist. Das Befestigungsmittel dient dazu, die Vorrichtung in dem Bereich des Patienten zu befestigen, in welchem das Implantat eingebracht werden soll.

Der vorliegenden Erfindung liegt die Aufgabe zugrunde, ein einfach zu handhabendes Hilfsmittel zur Durchmesserreduktion der Löcher in Bohrschablonen bereitzustellen, das gleichzeitig ein zu tiefes Eindringen des Bohrers verhindert. Die Aufgabe wird durch eine Bohrerführung mit den Merkmalen von Anspruch 1 gelöst. Weitere bevorzugte Ausführungsformen sind Gegenstand der athängigen Ansprüche.

Durch die erfindungsgemässe Bohrerführung ist es möglich, mit einer Vielzahl von verschiedenen Bohrern in einer Bohrschablone zu arbeiten, ohne dass eine komplizierte Handhabung von kleinen Reduktionshülsen nötig ist. Gleichzeitig ist es möglich, die maximale Bohrtiefe festzulegen, in dem ein weiteres Eindringen des Bohrers durch eine Anschlagfläche auf der Oberseite eines wenigstens einen Anschlags oder auf der Oberseite des Griffteils verhindert wird. Der Chirurg kann durch einfaches Drehen der erfindungsgemässen Bohrerführung eine Führungshülse mit einem bestimmten Innendurchmesser und einer bestimmten Bohrtiefe in die Bohrschablone einführen. Anschliessend nimmt er die nächste Bohrerführung, die einen grösseren Innendurchmesser aufweist, und führt sie so in die Bohrschablone ein, dass er wieder eine identische Bohrtiefe wie mit der ersten Bohrerführung erhält. Dieser Vorgang wird so oft wiederholt, bis ein Loch mit gewünschtem Durchmesser und Tiefe erhalten wird. Im Normalfall reicht ein Set bestehend aus vier Bohrerführungen mit unterschiedlichen Innendurchmessern aus, um eine Standardimplantatbohrung zu erhalten.

Die erfindungsgemässe Bohrerführung umfasst einen Griffteil und wenigstens zwei Führungshülsen mit jeweils einem oberen Ende und einem unteren Ende. Der Griffteil weist eine Oberseite und eine Unterseite auf. Die wenigstens zwei Führungshülsen an der Unterseite des Griffteils stehen vor, d.h. sie sind auf der gleichen Seite des Griffteils angeordnet. Auf der Oberseite des Griffteils steht genau ein Anschlag vor, der eine Verlängerung einer der wenigstens zwei Führungshülsen in axialer Richtung ist. Ein solcher Anschlag hat eine Höhe von ein paar Millimetern, daher kann eine erfindungsgemässe Bohrerführung auch bei einer schwierigen Platzsituation im Patientenmund einfach zum Einsatz kommen. Die beiden Führungshülsen sind in ie einem Endbereich des Griffteils angeordnet.

Ein Anschlag, der auf der Oberseite des Griffteils vorsteht und in axialer Richtung eine Verlängerung einer Führungshülse ist oder die Oberseite des Griffteils unmittelbar beim oberen Ende der Führungshülse, dienen als Anschlagfläche für den Bohrer, d.h. sie stellen sicher, dass der Chirurg nicht zu tief bohrt. Dadurch werden Verletzungen von Nerven beim Bohren vermieden. Da die Höhe der Anschlagflächen des Anschlags verschieden zur Höhe der Anschlagfläche auf der Oberseite des Griffteils ist, hat der Operateur die Wahl zwischen verschiedenen Bohrtiefen.

Der Griffteil kann starr oder formbar sein. Ebenso kann er flach, halbrund oder rund sein. Um eine bessere Sterilisierbarkeit zu gewährleisten, ist die Oberfläche bevorzugt glatt. Die Länge der erfindungsgemässen Bohrerführung ist derart gewählt, dass die potentiellen Bohrstellen im Kieferbereich erreicht werden und dabei der Griffteil in der Regel ausserhalb der Mundhöhle gefasst werden kann. Das bevorzugte Griffteil der erfindungsgemässen Bohrerführung hat eine Länge zwischen 5 und 12 cm.

Die Unterseite des Griffteils ist zumindest teilweise dazu bestimmt, auf einer Bohrschablone aufzuliegen. Dadurch erhält der Zahnarzt während dem Bohren noch eine zusätzliche Stabilität, so dass der Bohrer nicht rutschen kann. Bevorzugt liegt dabei dieser Auflageteil unmittelbar zur Führungshülse benachbart auf der Bohrschablone auf. Dies kann beispielsweise die radial nach aussen gehende Fortsetzung der Führungshülse am Griffteil sein oder aber auch ein Endstück des Griffteils.

Die Führungshülsen, die an der Unterseite des Griffteils vorstehen, weisen ein oberes und ein unteres Ende auf. Das obere Ende ist zu dem Griffteil gerichtet, während das untere Ende auf der von der Unterseite des Griffteils abgewandten Seite angeordnet ist. Es ist möglich die Führungshülsen separat zu fertigen und diese in den Griffteil einzufügen, wobei das obere Ende der Führungshülse mit dem Griffteil verbunden wird. Bevorzugt wird jedoch die Bohrerführung einstückig gefertigt, um eine bessere Sterilisierbarkeit sicherzustellen. Die Führungshülsen sind zylindrisch und weisen einen solchen Aussendurchmesser auf, dass sie passgenau in die Bohrlöcher der Bohrschablone respektive die entsprechenden Standardbohrhülsen passen. Der Innendurchmesser ist auf die Durchmesser der Bohrer abgestimmt.

Die Höhe der Führungshülsen und der Anschlagfläche des Anschlags respektive der Anschlagfläche der Oberseite des Griffteils bestimmen zusammen mit der Bohrergeometrie die Präzision und die Tiefe der Bohrung. Lange Führungshülsen erhöhen zwar die Präzision der Bohrerführung, gleichzeitig aber auch die Gesamthöhe der aufeinanderliegenden Teile der Bohrschablone, Bohrerführung und Bohrergerät. Befriedigende Ergebnisse werden mit Führungshülsen ab 5 mm Höhe im zylindrischen Bereich erreicht. Idealerweise beträgt die Höhe aber nicht mehr als 10 mm.

In einer bevorzugten Ausführungsform weist die Bohrerführung genau zwei Führungshülsen auf. Diese sind jeweils im Endbereich des Griffteils angeordnet. Dadurch sind die Führungshülsen in einem maximalen Abstand zueinander, was zur Folge hat, dass die zweite Führungshülse nicht den Einsatz der ersten Führungshülse behindert.

Es ist vorgeschen, dass auf der Oberseite des Griffteils genau ein Anschlag vorsteht, der in axialer Richtung eine Verlängerung einer der zwei Führungshülsen ist. Hier hat der Operateur demnach die Auswahl zwischen zwei Bohrtiefen. Entweder wählt er die Seite der Bohrerführung, die eine Führungshülse und einen Anschlag aufweist, oder aber er wählt die Seite, die eine Führungshülse aufweist und bei der als Anschlagfläche die bei oberen Ende dieser Führungshülse liegende Oberseite des Griffteils dient. Im ersten Fall wird der Bohrer weniger tief in den Knochen eindringen als beim zweiten Fall, da die Anschlagfläche des Anschlags früher auf den Bohrer trifft als die Anschlagfläche des Griffteils. Der Operateur kann damit auf einfache Weise die Bohrtiefe festlegen, ohne dass an dem Instrument, d.h. der Bohrerführung, vor dem Einsatz noch durch Schrauben oder Drehen eine bestimmte Einstellung eingerichtet werden muss. Die beiden Führungshülsen weisen einen identischen Innendurchmesser auf. Dieser wird vorzugsweise ausgewählt aus der Gruppe bestehend aus 2.2 mm, 2.8 mm, 3.5 mm und 4.2 mm.

Instrumente in der dentalen Implantologie müssen im besonderen Masse die Dimensionen und die Physiologie im Patientenmund berücksichtigen. Werden zwei Führungshülsen über ein Griffteil verbunden, sollten sie derart angeordnet sein, dass das zweite Ende der Bohrerführung, das sich nicht in der Bohrschablone befindet, nicht den Einsatz der in der Bohrschablone einzusetzenden ersten Führungshülse behindert. Es ist von Vorteil, wenn der Griffteil abgewinkelt ist. In einer bevorzugten Ausführungsform sind beispielsweise beide Enden des Griffteils s-förmig, sodass das Griffteil eine brückenartige Form aufweist. Dadurch hat die Zunge des Patienten darunter ausreichend Platz und wird durch das darüber liegenden Griffteil von der Bohrstelle ferngehalten. Zudem ist die Bohrerführung vorzugsweise derart geformt, dass sie sich auch im teilbezahnten Kiefer problemlos an jeder Stelle einsetzen lässt. Daher sollte die s-förmige Abwinkelung im Griffteil möglichst nahe bei der Führungshülse liegen. Gleichzeitig muss über der Führungshülse ausreichend Platz für das Kopfstück der gebräuchlichen Dentalbohrvorrichtungen bleiben. Die Führungshülsen der vorliegenden Erfindung stehen vorzugsweise parallel zueinander am Griffteil, weil dadurch die Gesamthöhe der Bohrerführung kleiner bleibt. Es ist jedoch auch eine Abwinkelung des Griffteils denkbar, wobei diese Abwinkelung vorzugsweise im mittleren Teil des Griffteils angeordnet ist. Durch eine solche Abwinkelung stehen die Führungshülsen ebenfalls abgewinkelt zueinander.

Bei der Verwendung von mehreren erfindungsgemässen Bohrerführungen kann eine Kodierung verwendet werden. Die häufig auf den Bohrern verwendeten Farbcodes können für die Bohrerführungen übernommen und an geeigneter Stelle, etwa am Griffteil nahe der entsprechenden Führungshülse, aufgebracht werden. Ebenso kann der Operateur durch eine Kodierung über die Bohrtiefe, d.h. über die Höhe des Anschlags informiert werden. Alternativ kann die Führungshülse selbst in der entsprechenden Farbe eingefärbt werden.

Operationsinstrumente in der Chirurgie müssen zuverlässig gereinigt und sterilisiert werden können. Es werden deshalb einteilige Bohrerführungen mit glatten, zugänglichen Oberflächen bevorzugt. Besonders bevorzugt werden einteilige einstückige Bohrerführungen, da sie auch keine Verbindungsstellen aufweisen. Mehrstückige Bohrerführungen, deren Verbindungsstellen keine Ritzen aufweisen und einfach zu reinigen sind, sind jedoch ebenfalls denkbar. Ebenfalls vorteilhaft ist es während einer Behandlung Operationsinstrumente verwenden zu können, die weder verstellt, umgebaut noch ausgetauscht werden müssen. Die erfindungsgemässe Bohrerführung werden diesen Anforderungen gerecht. Sie weisen keine Hinterschnitte auf und lassen sich leicht sterilisieren.

Die bevorzugten Materialen, aus denen die Bohrerführung der vorliegenden Erfindung gefertigt sind, umfassen rostfreien Stahl, Titan oder andere in der Chirurgie gängige Metalllegierungen. Um die Langlebigkeit der Bohrerführung zu erhöhen, können Teile der Bohrerführung, wie etwa die Führungshülsen, zusätzlich mit oberflächenhärtenden Verfahren behandelt werden. Ein zweckmässiges Verfahren zur Härtung von rostfreiem Stahl ist Kolsterisieren. In einer Ausführungsform ist das Material so gewählt, dass der Griffteil von Hand biegbar ist, so dass der Operateur ihn so formen kann, dass er möglichst optimal den räumlichen Gegebenheiten im Mund angepasst wird.

Die erfindungsgemässe Bohrerführung kann in Sets zusammen mit Bohrschablonen zur Verfügung gestellt werden. Die Bohrschablonen können wahlweise Standardbohrhülsen enthalten. Dabei sollte der Aussendurchmesser der Führungshülsen der Bohrerführung passend zum Innendurchmesser der Bohrlöcher der Bohrschablone respektive dem Innendurchmesser der Standardbohrhülse sein. Ebenso ist es möglich, in einem Set mehrere Bohrerführungen, die jeweils unterschiedliche Innendurchmesser aufweisen, zur Verfügung zu stellen. Idealerweise umfasst ein solches Set vier Bohrerführungen, wobei jede Bohrerführung zwei Führungshülsen mit identischem Innendurchmesser und einen Anschlag aufweist, der eine Verlängerung einer Führungshülse in axialer Richtung ist. Besonders bevorzugt weisen die Führungshülsen der ersten Bohrerführung einen Innendurchmesser von 2.2 mm, die Führungshülsen der zweiten Bohrerführung einen Innendurchmesser von 2.8 mm, die Führungshülsen der dritten Bohrerführung einen Innendurchmesser von 3.5mm und die Führungshülsen der vierten Bohrerführung einen Innendurchmesser von 4.2 mm auf.

Die erfindungsgemässe Bohrerführung wird nachfolgend im Detail anhand von bevorzugten Ausführungsformen in den Figuren 1 bis 4 weiter erläutert.
Fig. 1 zeigt eine erfindungsgemässe Bohrerführung mit zwei Führungshülsen und einem Anschlag.
Fig. 2a und 2b zeigen Ausschnitte der in Figur 1 gezeigten Bohrerführung
Fig. 3a und 3b zeigen die in Figur 1 gezeigte Bohrerführung in einer Bohrschablone
Fig. 4 zeigt eine Set mit vier verschiedenen Bohrführungen.

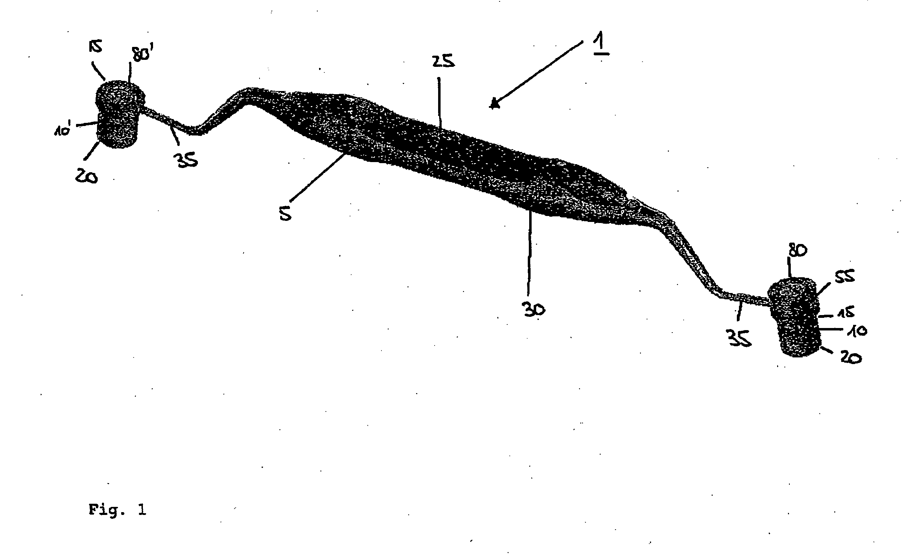

In Figur 1 wird eine Bohrerführung 1 zur Verwendung im Dentalbereich beschrieben. Die Bohrerführung 1 weist einen einteiligen Griffteil 5 und wenigstens zwei Führungshülsen 10, 10' mit jeweils einem oberen zum Griffteil gerichteten Ende 15 und einem unten vom Griffteil abgewandten Ende 20 auf. Der Griffteil weist eine Oberseite 25 und eine Unterseite 30 auf, wobei ein Teil 35 der Unterseite 30 dazu bestimmt ist, auf der Bohrschablone aufzuliegen. Dieser Teil 35, der in direkter Nachbarschaft zu der Führungshülse 10 steht, gewährleistet den Sitz der Bohrerführung auf der Bohrschablone. Die wenigstens zwei Führungshülsen 10 stehen an der Unterseite 30 des Griffteils vor und sind jeweils am Ende des Griffteils angeordnet. Dadurch, dass die Führungshülse gleichgerichtet sind, d.h. an der gleichen Seite des Griffteils angeordnet sind, ist es möglich, für die Zunge im Mundraum möglichst viel Raum zu lassen und dem Patienten einen angenehmen Öffnungswinkel des Mundes während des Bohrvorgangs zu erlauben. Damit die Bohrerführung eingesetzt in die Bohrschablone insgesamt nicht zu weit in den Mundraum wegsteht und dabei eine nachteilige Gesamthöhe erreicht, weist das Griffteil vorzugsweise eine doppelte Abwinkelung oder einen s-förmigen Abschnitt auf, so dass die Bohrerführung eine brückenartige Form hat. Die s-förmige Abwinkelung ist dabei möglichst nahe bei der Führungshülse. Auf der Oberseite 35 des Griffteils steht ein Anschlag 55 vor, der eine Verlängerung einer der zwei Führungshülsen 10 in axialer Richtung ist. Die Oberseite dieses Anschlags dient dazu als Anschlagfläche 80 für den Bohrer zu wirken, um so ein zu tiefes Eindringen in den Knochen zu verhindern. Auf der Oberseite 25 des Griffteils, der der zweiten Führungshülse 1'' gegenüberliegt, steht kein Anschlag vor. Hier dient die Oberseite des Griffteils als Anschlagfläche 80' für den Bohrer. Damit wird je nach dem, welche Führungshülse in die Bohrschablone eingeführt wird, eine unterschiedliche Bohrtiefe festgelegt.

Die in Figur 1 gezeigte Bohrerführung ist einstückig gefertigt und weist keinerlei Hinterschneidungen auf. Durch die Einstückigkeit gibt es keine Verbindungen zwischen einzelnen Komponenten, die es möglicherweise schwerer machen, die Bohrerführung zu sterilisieren. Es ist jedoch auch denkbar, dass die Bohrerführung gemäss Figur 1 mehrstückig gefertigt werden, indem die Führungshülsen separat hergestellt und mit dem Griffteil verbunden werden.

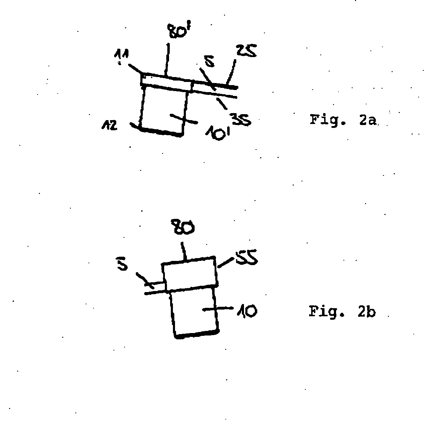

Figuren 2a und 2b zeigen einen Ausschnitt der Bohrerführung gemäss Figur 1. In Figur 2a wird die zweite Führungshülse 10' gezeigt, die auf der Unterseite des Griffteils 5 angeordnet ist. Die Führungshülse 10' weist ein oberes Ende 11 und ein unteres Ende 12 auf. Das obere Ende 12 entspricht der Oberseite 25 des Griffteils und dient als Anschlagfläche 80' für den Bohrer. In Figur 2b wird die erste Führungshülse 10 gezeigt, die auf der Unterseite des Griffteils 5 angeordnet ist. Auf der Oberseite des Griffteils steht ein Anschlag 55 vor, der eine Verlängerung der Führungshülse 10 in axialer Richtung ist. Die Oberseite des Anschlags 55 dient als Anschlagfläche 80 für den Bohrer.

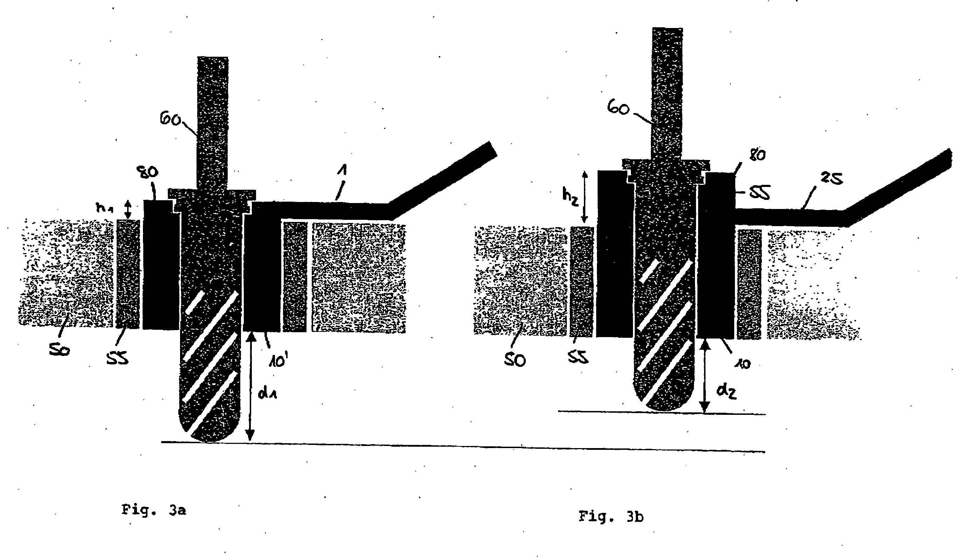

Figuren 3a und 3b zeigen einen Schnitt durch eine Bohrschablone 50 mit einer Standardbohrhülse 55, welche die Bohrerführung 1 gemäss Figur 1 enthält. In Figur 3a ist die Bohrerführung so in die Standardbohrhülse 55 eingeführt, dass der Bohrer 60 gegen die Oberseite des Griffteils, das heisst, die Anschlagfläche 80', schlägt, die damit als Bohrstopp wirkt. Der Bohrer 60 wird durch die Führungshülse 10 der Bohrerführung geführt. In Figur 3b ist die Bohrerführung so in die Standardbohrhülse eingeführt, dass der Bohrer 60 gegen die Anschlagfläche 80 des Anschlags 55 schlägt. Die Anschlagfläche 80 des Anschlags 55, der von der Oberseite 25 des Griffteils vorsteht und eine Verlängerung der Führungshülse 10 in axialer Richtung ist, ist von der Oberseite der Bohrschablone in der Höhe h2 beabstandet. Die Anschlagfläche 80', die der Oberseite des Griffteils entspricht, ist in der Höhe h1 von der Oberseite der Bohrschablone beabstandet. Die Differenz h2 - h1 entspricht dem Unterschied der Bohrtiefe d2 - d1, der erhalten wird, je nach dem, ob die Führungshülse ohne Anschlag oder die Führungshülse mit Anschlag beim Bohrern eingesetzt wird.

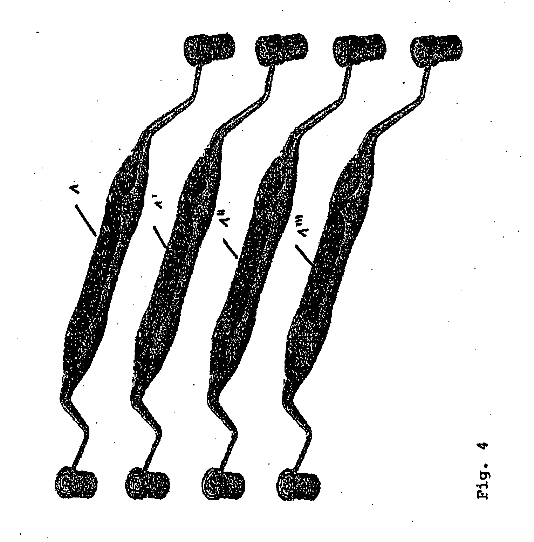

Figur 4 ein Set mit vier erfindungsgemässen Bohrerführungen. Die Bohrerführungen 1, 1', 1'' und 1''' unterscheiden sich dadurch, dass jede Bohrerführung Führungshülsen mit einem unterschiedlichen Innendurchmesser aufweist. Das heisst, die Führungshülsen der ersten Bohrerführung 1 weisen einen identischen Innendurchmesser auf, unterscheiden sich jedoch dadurch, dass sich auf der Oberseite des Griffteils auf der einen Seite ein Anschlag befindet, der eine Verlängerung einer Führungshülse in axialer Richtung ist. Mit der ersten Bohrerführung kann damit der Operateur mit einem bestimmten Bohrerdurchmesser bohren, er hat jedoch zwei Bohrtiefen zur Auswahl. Jede dieser Bohrerführungen 1, 1', 1'' und 1''' weisen zwei Führungshülsen mit identischem Innendurchmesser und einem Anschlag auf, der eine Verlängerung einer Führungshülse in axialer Richtung ist. Die Anschläge sämtlicher Bohrerführungen sind gleich hoch. Damit kann der Operateur die Bohrtiefe konstant halten und den Bohrdurchmesser kontinuierlich erweitern. Besonders bevorzugt weisen die Führungshülsen der ersten Bohrerführung 1 einen Innendurchmesser von 2.2 mm, die Führungshülsen der zweiten Bohrerführung 1' einen Innendurchmesser von 2.8 mm, die Führungshülsen der dritten Bohrerführung 1'' einen Innendurchmesser von 3.5mm und die Führungshülsen der vierten Bohrerführung 1''' einen Innendurchmesser von 4.2 mm auf.

## Patentansprüche

1. Bohrerführung (1) für verschiedene Bohrtiefen zur Verwendung im Dentalbereich umfassend einen Griffteil (5) und wenigstens zwei am Griffteil angeordnete Führungshülsen (10), wobei der Griffteil (5) eine Oberseite (25) und eine Unterseite (30) aufweist, und die Unterseite (30) des Griffteils dazu bestimmt ist, zumindest teilweise auf einer Bohrschablone aufzuliegen, wobei die wenigstens zwei Führungshülsen (10) auf der Unterseite (30) des Griffteils vorstehen, wobei in beiden Endbereichen des Griffteils (5) je eine Führungshülse (10) angeordnet ist, **dadurch gekennzeichnet, dass** auf der Oberseite (25) des Griffteils genau ein Anschlag (55) vorsteht, der in axialer Richtung eine Verlängerung einer der zwei Führungshülsen (10) ist, und dass der Innendurchmesser der wenigstens zwei Führungshülsen (10) identisch ist.

2. Bohrerführung (1) nach Anspruch 1, **dadurch gekennzeichnet, dass** die Bohrerführung (1) einstückig ist.

3. Bohrerführung (1) nach Anspruch 1, **dadurch gekennzeichnet, dass** die Bohrerführung (1) mehrstückig ist.

4. Bohrerführung nach einem der Ansprüche 1 bis 3, **dadurch gekennzeichnet, dass** der Griffteil in der Nähe der Führungshülse einen s-förmigen Abschnitt aufweist.

5. Bohrerführung (1) nach einem der vorangehenden Ansprüche, **dadurch gekennzeichnet, dass** die Führungshülsen (10) in axialer Richtung parallel zueinander stehen.

6. Bohrerführung (1) nach einem der vorangehenden Ansprüche, **dadurch gekennzeichnet, dass** die Führungshülsen (10) in axialer Richtung angewinkelt zueinander stehen.

7. Bohrerführung (1) gemäss einem der vorangehenden Ansprüche aus rostfreiem Stahl.

8. Bohrerführung (1) nach einem der vorangehenden Ansprüche, **dadurch gekennzeichnet, dass** der Innendurchmesser der zwei Führungshülsen eine Grösse ausgewählt aus der Gruppe von 2.2 mm, 2.8 mm, 3.5 mm und 4.2 mm aufweist.

9. Set, umfassend eine Bohrschablone (50) mit Bohrlöchern und eine Bohrerführung (1) nach einem der Ansprüche 1 bis 8, **dadurch gekennzeichnet, dass** der Aussendurchmesser der Führungshülsen (10) passend zum Innendurchmesser der Bohrlöcher der Bohrschablone ist.

10. Set, umfassend eine Bohrschablone mit Bohrlöchern enthaltend Standardbohrhülsen und eine Bohrerführung nach einem der Ansprüche 1 bis 8, **dadurch gekennzeichnet, dass** der Aussendurchmesser der Führungshülsen passend zum Innendurchmesser der in den Bohrlöchern der Bohrschablone enthaltenen Standardbohrhülsen ist.

11. Set, umfassend wenigstens zwei Bohrerführungen gemäss einem der Ansprüche 1 bis 8, **dadurch gekennzeichnet, dass** sämtliche Bohrerführungen Führungshülsen mit unterschiedlichen Innendurchmessern aufweisen,

12. Set nach Anspruch 11, umfassend vier Bohrerführungen gemäss einem der Ansprüche 1 bis 8.

## Claims

1. Drill guide (1) for various drilling depth for use in the dental field comprising a grip part (5) and at least two guide sleeves (10) arranged on the grip part, wherein the grip part (5) has a top face (25) and an underside (30), and the underside (30) of the grip part is intended to bear at least partially on a drill jig, wherein the at least two guide sleeves (10) protrude from the underside (30) of the grip part, wherein a guide sleeve (10) is arranged in both end zones of the grip part (5), **characterized in that** exactly one stop (55), which is an axial continuation of one of the two guide sleeves (10), protrudes from the top face (25) of the grip part, and **in that** the internal diameter of the at least two guide sleeves (10) is identical.

2. Drill guide (1) according to claim 1, **characterized in that** the drill guide (1) is made in one piece.

3. Drill guide (1) according to claim 1, **characterized in that** the drill guide (1) is made up of several pieces.

4. Drill guide (1) according to one of claims 1 to 3, **characterized in that** the grip has an S-shaped portion near the guide sleeve.

5. Drill guide (1) according to one of the preceding claims, **characterized in that** the guide sleeves (10) are parallel to one another in the axial direction.

6. Drill guide (1) according to one of the A18484EP/06.07.2015 preceding claims, **characterized in that** the guide sleeves (10) are at an angle to one another in the axial direction.

7. Drill guide (1) according to one of the preceding claims, made of stainless steel.

8. Drill guide (1) according to one of the preceding claims, **characterized in that** the internal diameter of the two drill guides has a dimension selected from the group of 2.2 mm, 2.8 mm, 3.5 mm and 4.2 mm.

9. Set comprising a drill jig (50) with drill holes and a drill guide (1) according to one of claims 1 to 8, **characterized in that** the external diameter of the guide sleeves (10) matches the internal diameter of the drill holes of the drill jig.

10. Set comprising a drill jig with drill holes containing standard drill sleeves, and a drill guide according to one of claims 1 to 8, **characterized in that** the external diameter of the guide sleeves matches the internal diameter of the standard drill sleeves contained in the drill holes of the drill jig.

11. Set comprising at least two drill guides according to one of claims 1 to 8, **characterized in that** all the drill guides have guide sleeves of different internal diameters.

12. Set according to claim 11, comprising four drill guides according to one of claims 1 to 8.

## Revendications

1. Guide de forage (1) pour différentes profondeurs de forage pour l'utilisation dans le domaine dentaire comprenant une partie de préhension (5) et au moins deux douilles de guidage (10) disposées sur la partie de préhension, la partie de préhension (5) présentant une face supérieure (25) et une face inférieure (30), et la face inférieure (30) de la partie de préhension étant prévue pour s'appliquer au moins en partie sur un gabarit de forage, les au moins deux douilles de guidage (10) faisant saillie sur le côté inférieur (30) de la partie de préhension, une douille de guidage (10) étant disposée à chacune des deux zones d'extrémité de la partie de préhension (5), **caractérisé en ce que**, sur la face supérieure (25) de la partie de préhension exactement une butée (55) fait saillie en prolongement d'une des deux douilles de guidage (10) dans la direction axiale, et **en ce que** le diamètre intérieur des au moins deux douilles de guidage (10) est identique.

2. Guide de forage (1) selon la revendication 1, **caractérisé en ce que** le guide de forage (1) est réalisé d'une seule pièce.

3. Guide de forage (1) selon la revendication 1, **caractérisé en ce que** le guide de forage (1) est constitué de plusieurs pièces.

4. Guide de forage selon l'une des revendications 1 à 3, **caractérisé en ce que** la partie de préhension présente une portion en forme de S à proximité de la douille de guidage.

5. Guide de forage (1) selon l'une des revendications précédentes, **caractérisé en ce que** les douilles de guidage (10) sont parallèles l'une à l'autre dans la direction axiale.

6. Guide de forage (1) selon l'une des revendications précédentes, **caractérisé en ce que** les douilles de guidage (10) forment un angle l'une par rapport à l'autre dans la direction axiale.

7. Guide de forage (1) selon l'une des revendications précédentes, constitué d'acier inoxydable.

8. Guide de forage (1) selon l'une des revendications précédentes, **caractérisé en ce que** le diamètre intérieur des deux douilles de guidage présente une dimension sélectionnée dans le groupe de 2.2 mm, 2.8 mm, 3.5 mm et 4.2 mm.

9. Kit comprenant un gabarit de forage (50) avec des trous de forage et un guide de forage (1) selon l'une des revendications 1 à 8, **caractérisé en ce que** le diamètre extérieur des douilles de guidage (10) est adapté au diamètre intérieur des trous de forage du gabarit de forage.

10. Kit comprenant un gabarit de forage avec des trous de forage contenant des douilles de forage standard et un guide de forage selon l'une des revendications 1 bis 8, **caractérisé en ce que** le diamètre extérieur des douilles de guidage est adapté au diamètre intérieur des douilles de forage standard contenues dans les trous de forage du gabarit de forage.

11. Kit comprenant au moins deux guides de forage selon l'une des revendications 1 à 8, **caractérisé en ce que** tous les guides de forage présentent des douilles de guidage avec des diamètres intérieurs différents.

12. Kit selon la revendication 11 comprenant quatre guides de forage selon l'une des revendications 1 à 8.
